# EUROPEAN PATENT APPLICATION

(11) **EP 4 617 264 A1**
(43) Date of publication of application: **17.09.2025**
(21) Application number: 23889025.5
(22) Date of filing: 01.11.2023
(51) Int. Cl.: C07D 401/04, A61K 31/454, A61P 35/00, A61P 37/06, A61P 29/00

(54) **ISOINDOLINONE DERIVATIVE HAVING ARYLCYCLOALKYLAMIDE STRUCTURE, AND USE THEREOF**

(30) Priority: 08.11.2022 KR 20220148246
(71) Applicant: Oncord Bio Inc, Seoul 04050 (KR); Korea Research Institute of Chemical Technology, Daejeon 34114 (KR)
(72) Inventor: HWANG, Jong Yeon, Daejeon 34114 (KR); LEE, Soon Im, Seoul 04050 (KR); KOO, Jae Kyung, Seoul 04050 (KR); CHOE, Seong Choon, Seoul 04050 (KR); CHO, Sung Yun, Daejeon 34114 (KR); HA, Jae Du, Daejeon 34114 (KR); KIM, Pil Ho, Daejeon 34114 (KR); KIM, Hyun Jin, Daejeon 34114 (KR); CHO, Yong Hee, Daejeon 34114 (KR); AKSHAY, Diw, Daejeon 34114 (KR)
(74) Representative: Kraus & Lederer PartGmbB
(86) International application number: PCT/KR2023/017237
(87) International publication number: WO 2024/101763

(57) **Abstract**

The present invention relates to an isoindolinone derivative compound having a glutarimide mother nucleus, and an application thereof, and more specifically, to an isoindolinone derivative compound in which a glutarimide mother nucleus is substituted with phenylcyclopropane having a thalidomide analog structure. The compound of chemical formula 1 according to the present invention specifically binds to the CRBN protein and is involved in functions thereof. Thus, the compound of the present invention may be beneficially used in the prevention or treatment of leprosy, chronic graft versus host disease, inflammatory diseases, or cancer caused by actions of the CRBN protein.

## Description

### Technical Field

The present disclosure relates to a phenylcycloalkyl-substituted isoindolinone having a glutarimide moiety and a use thereof. More specifically, the present disclosure relates to a phenylcycloalkyl-substituted isoindolinone having a glutarimide moiety, which exhibits prophylactic or therapeutic effects on leprosy, chronic graft-versus-host disease, inflammatory diseases, or cancer.

### Background Art

Thalidomide is a racemic compound sold under the trademark THALOMID^{®} and the chemical name α-(N-phthalimido)glutarimide or 2-(2,6-dioxo-3-piperidinyl)-1H-isoindole-1,3(2H)-dione. Thalidomide was originally developed to treat morning sickness but was discontinued due to its teratogenic effects. Thalidomide is currently approved in the United States for the treatment of erythema nodosum leprosum in humans (Korean Patent No. 10-0671366).

In addition, thalidomide has been reported to be used in patients with leprosy, chronic graft-versus-host disease, rheumatoid arthritis, sarcoidosis, certain inflammatory skin diseases, and inflammatory bowel disease. It has also been reported that thalidomide can be combined with other drugs to treat ischemia/reperfusion related to cardiac and cerebral artery occlusion (U.S. Patent No. 5,643,915).

More recently, thalidomide has been used to treat certain types of cancer, including refractory multiple myeloma, brain cancer, melanoma, breast cancer, colon cancer, mesothelioma, and renal cell carcinoma. Thalidomide has also been reported to be used to prevent the manifestation of chronic cardiomyopathy induced by doxorubicin in rats. Other reports on the use of thalidomide in cancer treatment include its coadministration with carboplatin in the treatment of glioblastoma multiforme. It has also been reported that thalidomide is used as an analgesic in the treatment of astrocytoma (Costa, P. T. et al., Blood, 92(Suppl 1, Pt 2), 235b, 1998; Marx, G. M. et al., Proc Am Soc Clin Oncol., 454a, 1999; Singhal, S. et al., N Engl J Med., 341(21), 1565-1571, 1999; Zwart, D., Arzneimittelforschung, 16(12), 1688-1689, 1966).

Furthermore, thalidomide has been widely used for the prevention or treatment of various diseases such as lupus nephritis, fibromyalgia, schizophrenia, central nervous system disorders, diabetes, and inflammatory diseases. However, due to its fatal side effect of causing birth defects when taken by pregnant women, it was withdrawn from the market at the end of 1961.

Active research is underway to develop derivatives that retain the various physiological benefits of thalidomide while eliminating its serious side effects.

The degradation of intracellular proteins mainly occurs via the ubiquitin-proteasome system (UPS). Proteins called E1, E2, and E3 ligases transfer ubiquitin (Ub), composed of 76 amino acids, to the substrate protein to be degraded, causing polyubiquitination, which is then recognized and degraded by the 26S proteasome.

Lenalidomide and pomalidomide, which are derivatives of thalidomide, known as immunomodulatory drugs (IMiDs), bind to an E3 ligase called cereblon (CRBN) to induce the degradation of zinc finger transcription factors ikaros (IKZF1) and aiolos (IKZF3), thus finding applications as therapeutics for multiple myeloma. In particular, lenalidomide degrades CK-1α and is also used as a therapeutic agent for patients with 5q-deletion myelodysplastic syndrome (5q-del-MDS), and is one of the best-selling anticancer drugs worldwide.

GSPT1 is a GTPase that forms a complex with eRF1 upon GTP binding and binds to the stop codon of mRNA. When GTP is hydrolyzed to GDP, GSPT1 dissociates from eRF1, thereby participating in protein cleavage by eRF1 (Cell Reports, 2014, 8, 59-65).

It is known that knockdown of GSPT1 reduces the phosphorylation of 4E-BP1 and kinase S6K1, thereby inhibiting mTOR activity and inducing G1 arrest (Molecular and Cellular Biology, 2007, 27, 5619).

GSPT1 is overexpressed in colon cancer cell lines (HCT116) and is involved in cell growth and migration. It was confirmed that knockdown of GSPT1 suppresses the expression of c-myc, survivin, and Bcl2L15, thereby inducing apoptosis (Biomed. Pharmacother. 2015, 74, 138-144).

In addition, Celgene reported that the CC-885 compound, derived from lenalidomide, degrades a novel protein, GSPT1, and exhibits high cytotoxicity in various hematological cancer cells. Through optimization studies, the CC-90009 compound was derived and is currently in Phase 1 clinical trials in patients with R/R-AML (Nature, 2016, 14, 252; Blood, 2021).

Development was made of novel piperidine-2,6-dione-based thalidomide derivatives that retain the physiological activity of thalidomide while eliminating its side effects, and it was confirmed that the derivative compounds promote the degradation of GSPT1 protein and Aiolos. Moreover, the developed compounds showed sufficient cytotoxicity in cancer cells, and in particular, urea derivative-substituted compounds and triazine derivative compounds exhibited superior cytotoxic activity against cancer cells (Korean Patent No. 10-2020-0054046 A).

In addition, 5-substituted isoindoline compounds have been reported to exhibit not only TNF-α inhibition and IL-2 production effects, but also antiproliferative effects against various cancer cells including prostate, colon, pancreatic, and breast tumors (Korean Patent No. 10-2011-0019761 A). It has also been reported that isoindoline compounds exert effects on various diseases including cancer by controlling angiogenesis, inhibiting the production of specific cytokines such as TNF-α, and stimulating the production of specific cytokines such as IL-10 (Korean Patent No. 10-1696938).

Furthermore, a method for predicting and treating various diseases including cancer by administering compounds having isoindolinone and glutarimide have been reported (Korean Patent No. 10-2018-0095094 A), and compounds having aminoamide linkers have been reported to act as modulators of various protein activities including cytokines, TNF-α, and GSPT1, thereby being effective in treating various diseases such as inflammatory diseases and cancer (Australian Patent No. 2019284608 A).

Leading to the present disclosure, intensive and thorough research conducted by the present inventors resulted in the finding that the compound represented by Chemical Formula 1 of the present disclosure, which has a phenylcycloalkyl-substituted glutarimide group and an isoindolinone structure, exhibits excellent biological activity against cancer cells compared to compounds disclosed in prior patents.

### Disclosure of Invention

### Technical Problem

The present inventors have completed the present disclosure by developing phenylcyclopropane-substituted isoindolinone derivative compounds having a glutarimide moiety and evaluating the compounds for prophylactic or therapeutic effects on leprosy, chronic graft-versus-host disease, inflammatory diseases, or cancer, and by evaluating the activity thereof.

Accordingly, the present disclosure aims to provide a compound represented by the following Chemical Formula 1 or a pharmaceutically acceptable salt thereof.

### Solution to Problem

The present disclosure relates to a compound represented by the following Chemical Formula 1, a racemate, an optical isomer, or a pharmaceutically acceptable salt thereof: 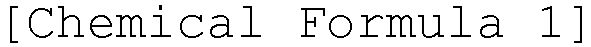 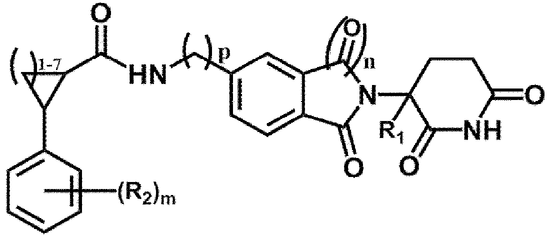 wherein,
m is an integer from 0 to 3;
n is an integer of 0 or 1;
p is an integer of 1 or 2;
R₁ is hydrogen or deuterium (D);
R₂ is independently at least one substituent selected from the group consisting of hydrogen, halogen, hydroxy, carboxylic acid, C₁₋₅ alkylcarboxyl, amino, acetamido, sulfonic acid, C₁₋₅ alkylsulfonic acid, nitro, mono- or di-(C₁₋₅ alkyl) amino, C₁₋₅ alkyl, C₁₋₃ alkoxy, halo-C₁₋₃ alkyl, and halo-C₁₋₃ alkoxy.

In addition, the present disclosure relates to a compound represented by the following Chemical Formula 2, a racemate, an optical isomer, a stereoisomer, or a pharmaceutically acceptable salt thereof: 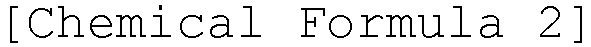 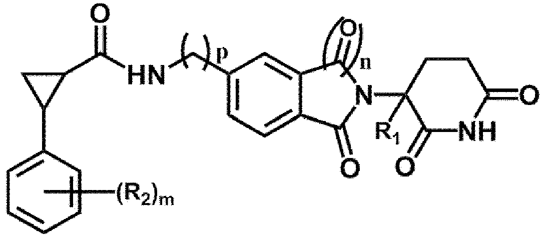 wherein,
m is an integer from 0 to 3;
p is an integer of 1 or 2;
R₁ is hydrogen or deuterium (D);
R₂ is independently at least one substituent selected from the group consisting of hydrogen, halogen, hydroxy, carboxylic acid, C₁₋₅ alkylcarboxyl, amino, acetamido, sulfonic acid, C₁₋₅ alkylsulfonic acid, nitro, mono- or di-(C₁₋₅ alkyl) amino, C₁₋₅ alkyl, C₁₋₃ alkoxy, halo-C₁₋₃ alkyl, and halo-C₁₋₃ alkoxy.

Concrete examples of the compound of Chemical Formula 1 in the present disclosure include:
(1S,2S)-N-((2-(2,6-dioxopiperidin-3-yl)-1-oxoisoindolin-5-yl)methyl)-2-phenylcyclopropane-1-carboxamide (Compound 1);
(1R,2R)-N-((2-(2,6-dioxopiperidin-3-yl)-1-oxoisoindolin-5-yl)methyl)-2-phenylcyclopropane-1-carboxamide (Compound 2);
(1R,2R)-2-(4-chlorophenyl)-N-((2-(2,6-dioxopiperidin-3-yl)-1-oxoisoindolin-5-yl)methyl)cyclopropane-1-carboxamide (Compound 3);
(1R,2S)-N-((2-(2,6-dioxopiperidin-3-yl)-1-oxoisoindolin-5-yl)methyl)-2-phenylcyclopropane-1-carboxamide (Compound 4);
(1R,2R)-2-(4-chlorophenyl)-N-((2-(2,6-dioxopiperidin-3-yl)-1-oxoisoindolin-5-yl)methyl)cyclopropane-1-carboxamide (Compound 5);
(1R,2R)-2-(3-chlorophenyl)-N-((2-(2,6-dioxopiperidin-3-yl)-1-oxoisoindolin-5-yl)methyl)cyclopropane-1-carboxamide (Compound 6);
(1S,2S)-N-((2-(2,6-dioxopiperidin-3-yl)-1-oxoisoindolin-5-yl)methyl)-2-phenylcyclopropane-1-carboxamide (Compound 7);
(1R,2R)-N-((2-(2,6-dioxopiperidin-3-yl)-1-oxoisoindolin-5-yl)methyl)-2-(4-fluorophenyl)cyclopropane-1-carboxamide (Compound 8);
(1R,2R)-N-((2-(2,6-dioxopiperidin-3-yl)-1-oxoisoindolin-5-yl)methyl)-2-(3-fluorophenyl)cyclopropane-1-carboxamide (Compound 9);
(1S,2S)-2-(3-chlorophenyl)-N-((2-(2,6-dioxopiperidin-3-yl)-1-oxoisoindolin-5-yl)methyl)cyclopropane-1-carboxamide (Compound 10);
N-((2-(2,6-dioxopiperidin-3-yl)-1-oxoisoindolin-5-yl)methyl)-2-(4-(trifluoromethyl)phenyl)cyclopropane-1-carboxamide (Compound 11);
(1S,2S)-N-((2-(2,6-dioxopiperidin-3-yl)-1-oxoisoindolin-5-yl)methyl)-2-(p-tolyl)cyclopropane-1-carboxamide (Compound 12);
2-(2,4-dichlorophenyl)-N-((2-(2,6-dioxopiperidin-3-yl)-1-oxoisoindolin-5-yl)methyl)cyclopropane-1-carboxamide (Compound 13); and
N-((2-(2,6-dioxopiperidin-3-yl)-1-oxoisoindolin-5-yl)methyl)-2-phenylcyclobutane-1-carboxamide (Compound 14).

In addition, the compound represented by Chemical Formula 1 of the present disclosure may be prepared by the methods illustrated in the following Reaction Schemes 1 and 2:

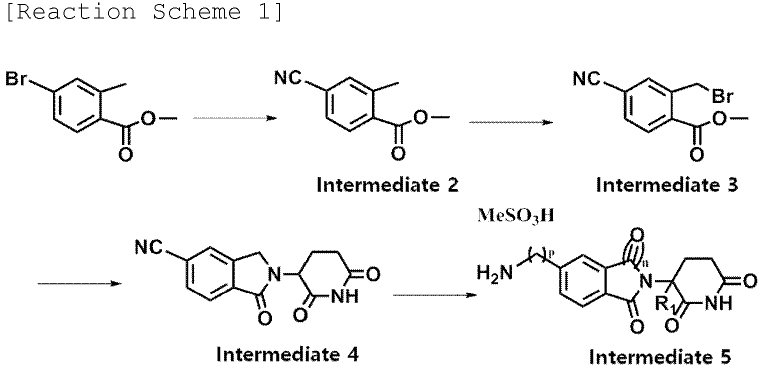

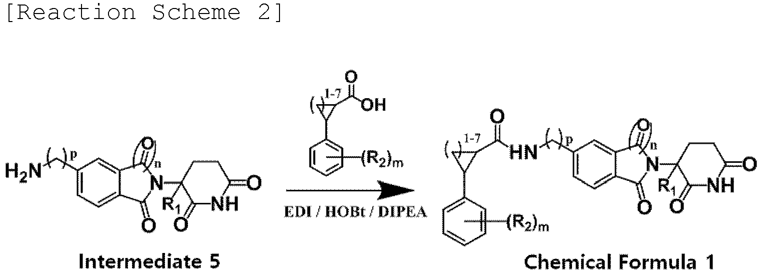

Reaction Scheme 2 illustrates a preparation method disclosed in Korean Patent No. 10-2011-0019761 A.

The method for preparing the compound represented by Chemical Formula 1 according to the present disclosure, as illustrated in the above preparation methods, should be understood as merely one example of a method for preparing the compound of the present disclosure. Any method capable of preparing the compound represented by Chemical Formula 1 being produced in the present disclosure is included in the present disclosure without limitation. In addition, the methods described in the specification of the present disclosure, as well as any modifications and alterations thereof that can be easily carried out by those of ordinary skill in the art, are also to be understood as falling within the scope of the present disclosure, as such modifications would be apparent to those skilled in the art.

Unless otherwise specified, the following terms used in the present disclosure have the meanings as defined below. Any undefined term shall be construed as having the meaning commonly understood by those skilled in the art.

As used herein, the term "halo" or "halogen" refers to fluorine (F), chlorine (Cl), bromine (Br), and iodine (I).

As used herein, the term "alkyl" refers to a straight or branched hydrocarbon group having a single bond. Examples include, but are not limited to, methyl, ethyl, and propyl.

As used herein, the term "alkoxy" refers to an oxygen group to which a saturated straight or branched hydrocarbon is bonded via a single bond. Examples include, but are not limited to, methoxy, ethoxy, and propoxy.

As used herein, the term "haloalkyl" refers to a substituted alkyl group as described above, wherein one or more hydrogen atoms on the alkyl group have been substituted with a halo group. Examples of such groups include, are not limited to, trifluoromethyl, difluoromethyl, trifluoroethyl, and the like.

As used herein, the term "haloalkoxy" refers to an alkyl-O- group wherein one or more hydrogen atoms on the alkyl group are substituted by halo groups. Examples include, but are not limited to, trifluoromethoxy.

The compound of Chemical Formula 1 of the present disclosure may include not only pharmaceutically acceptable salts thereof, but also all salts, hydrates, solvates, and prodrugs that can be prepared by conventional methods.

In addition, the compound of the present disclosure may contain one or more chiral carbon atoms and may exist in racemic form or optically active form. All such compounds and stereoisomers fall within the scope of the present disclosure.

Compounds that have the same molecular formula but differ in properties or in the order of bonding or spatial arrangement of atoms are referred to as "isomers." Isomers that differ in the spatial arrangement of atoms are referred to as "stereoisomers." Diastereomers are stereoisomers that are not enantiomers and have opposite configurations at one or more chiral centers. Stereoisomers that are non-superimposable mirror images of each other and contain one or more chiral centers are referred to as "enantiomers." When a compound has a chiral center, for example, when a carbon atom is bonded to four different groups, a pair of enantiomers is possible. One enantiomer may be described by its absolute configuration at the chiral center(s), in accordance with the Cahn-Ingold-Prelog R- and S-nomenclature rules, or by the direction in which it rotates the plane of polarized light, i.e., dextrorotatory or levorotatory (that is, (+)- or (-)-isomer, respectively). Chiral compounds may exist as individual enantiomers or as mixtures thereof. A mixture containing equal amounts of the two enantiomers is referred to as a "racemic mixture." The present disclosure includes mirror image isomers, partial stereoisomers, racemates, cis-isomers, trans-isomers, and mixtures thereof, whenever chemically feasible.

As used herein, the term "pharmaceutically acceptable salt" refers to a salt or complex of the compound of Chemical Formula 1 that exhibits desirable biological activity. Examples of such salts include, but are not limited to, acid addition salts formed with inorganic acids (e.g., hydrochloric acid, hydrobromic acid, sulfuric acid, phosphoric acid, nitric acid), and organic acids (e.g., acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and polygalacturonic acid). The compound may also be administered as a pharmaceutically acceptable quaternary salt known to those skilled in the art, particularly chloride, bromide, iodide, -O-alkyl, toluenesulfonate, methylsulfonate, sulfonate, phosphate, or carboxylate (e.g., benzoate, succinate, acetate, glycolate, maleate, malate, fumarate, citrate, tartrate, ascorbate, cinnamoate, mandeloate, and diphenylacetate).

The acid addition salts of the present disclosure can be prepared by conventional methods. For example, the derivative of Chemical Formula 1 may be dissolved in an organic solvent such as methanol, ethanol, acetone, dichloromethane, or acetonitrile, and an organic or inorganic acid may be added thereto to precipitate the salt, which may then be collected by filtration and dried. Alternatively, the solvent and an excess of acid may be subjected to vacuum distillation and then dried, followed by crystallization from the organic solvent.

In addition, metal salts may be prepared using a base to form pharmaceutically acceptable salts. Alkali metal or alkaline earth metal salts may be obtained, for example, by dissolving the compound in a solution of an excess of an alkali metal hydroxide or alkaline earth metal hydroxide, filtering the insoluble salt of the compound, and evaporating and drying the filtrate. In such cases, sodium, potassium, or calcium salts are suitable for pharmaceutical purposes. Moreover, the corresponding salts may be obtained by reacting the alkali metal or alkaline earth metal salts with an appropriate silver salt (e.g., silver nitrate).

The present disclosure also relates to a pharmaceutical composition including the compound represented by Chemical Formula 1 or a pharmaceutically acceptable salt thereof as an active ingredient for the prevention or treatment of leprosy, chronic graft-versus-host disease, inflammatory diseases, or cancer.

According to one experimental example of the present disclosure, it was confirmed that the compound binds to the CRBN (cereblon) protein and induces degradation of Ikaros/Aiolos and GSPT1. CRBN protein is a type of E3 ubiquitin ligase, and is known to bind with thalidomide and its analogs such as pomalidomide and lenalidomide to promote the attachment of ubiquitin to substrate proteins such as Ikaros/Aiolos and GSPT1.

The cancer may be selected from the group consisting of, but not limited to, breast cancer, colon cancer, lung cancer, small cell lung cancer, gastric cancer, liver cancer, hematologic cancer, bone cancer, pancreatic cancer, skin cancer, head or neck cancer, intraocular or cutaneous melanoma, uterine cancer, ovarian cancer, rectal cancer, anal cancer, colorectal cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, CNS tumors, primary CNS lymphoma, spinal tumors, brainstem glioma, and pituitary adenoma.

The pharmaceutical composition of the present disclosure may be formulated in a suitable form with a pharmaceutically acceptable carrier commonly used in the field. The term "pharmaceutically acceptable" refers to a composition that is physiologically acceptable and does not cause allergic or similar adverse reactions, such as gastrointestinal disorders or dizziness, when administered to humans. The composition may be formulated, according to conventional methods, into oral dosage forms such as powders, granules, tablets, capsules, suspensions, emulsions, syrups, or aerosols, as well as topical agents, suppositories, and sterile injectable solutions.

Carriers, excipients, and diluents that may be included in the composition include, but are not limited to, lactose, dextrose, sucrose, sorbitol, mannitol, xylitol, erythritol, maltitol, starch, gum arabic, alginates, gelatin, calcium phosphate, calcium silicate, cellulose, methylcellulose, microcrystalline cellulose, polyvinylpyrrolidone, water, methyl p-hydroxybenzoate, propyl p-hydroxybenzoate, talc, magnesium stearate, and mineral oil. When formulated, commonly used diluents or excipients such as fillers, stabilizers, binders, disintegrants, and surfactants may be used. Solid oral dosage forms may include tablets, pills, powders, granules, and capsules. These solid dosage forms are prepared by mixing at least one excipient, such as starch, microcrystalline cellulose, sucrose or lactose, or low-substituted hydroxypropylcellulose or hydroxypropyl methylcellulose, with the compound of the present disclosure. In addition to simple excipients, lubricants such as magnesium stearate and talc may also be used. Liquid oral dosage forms may include suspensions, internal solutions, emulsions, and syrups, which may contain not only commonly used aqueous diluents and liquid paraffin, but also various excipients such as wetting agents, sweeteners, flavoring agents, and preservatives. Dosage forms for parenteral administration may include sterile aqueous solutions, non-aqueous solvents, suspensions, emulsions, lyophilized formulations, and suppositories. The non-aqueous solvents or suspending agents may include injectable esters such as propylene glycol, polyethylene glycol, vegetable oils such as olive oil, or ethyl oleate. Bases for suppositories may include witepsol, macrogol, Tween 61, cacao butter, laurin butter, glycerol, and gelatin. To formulate a parenteral dosage form, the compound represented by Chemical Formula 1 or a pharmaceutically acceptable salt thereof may be mixed with water in combination with pharmaceutically acceptable excipients such as preservatives, stabilizers, hydrating agents, emulsifying agents, salts for osmotic pressure control, and/or buffering agents, and other therapeutically useful substances. The resulting solution or suspension may be prepared in unit dosage forms such as ampoules or vials.

The pharmaceutical composition may include the compound of Chemical Formula 1 and an excipient. The compound may be included in an amount of preferably 0.001 to 50% by weight, more preferably 0.001 to 40% by weight, and most preferably 0.001 to 30% by weight, based on the total weight of the composition.

The pharmaceutical composition comprising the compound of Chemical Formula 1 as an active ingredient may be administered to mammals, including rodents, livestock, and humans, through various routes. All known methods of administration may be employed, such as oral, rectal, intravenous, intramuscular, subcutaneous, intrauterine, intradural, or intracerebrovascular injection. The dosage may vary depending on the age, sex, weight of the subject, specific disease or pathological condition to be treated, severity of the condition, administration time, route of administration, absorption, distribution, and excretion rate of the drug, types of other drugs used, and the judgment of the prescribing physician. Determination of the dosage based on these factors is within the level of those skilled in the art. In general, the dosage may range from about 0.01 mg/kg/day to about 2000 mg/kg/day. A more preferable dosage is 1 mg/kg/day to 500 mg/kg/day. The dosage may be administered once daily or divided into multiple doses. The above dosage does not limit the scope of the present disclosure in any way.

### Advantageous Effects of Invention

The compound of Chemical Formula 1 according to the present disclosure specifically binds to the CRBN protein and is involved in the function thereof. Accordingly, the compound of the present disclosure can be advantageously used for the prevention or treatment of leprosy, chronic graft-versus-host disease, inflammatory diseases, or cancer through the action of CRBN protein.

### Brief Description of Drawings

FIG. 1 illustrates the degradation activity on GSPT1 and beta-actin after treatment of KG-1 cells with Compound 1 of the present disclosure for 6 hours.
FIG. 2 illustrates the inhibitory effect on cancer cell growth after treatment of H1155 cells with a compound of the present disclosure for 72 hours.

### Best Mode for Carrying out the Invention

Hereinafter, preferred embodiments of the present disclosure will be described in detail. However, the present disclosure is not limited to the embodiments described herein and may be embodied in other forms. Rather, the present disclosure is provided so that it is thorough and complete, and to fully convey the scope of the present disclosure to those skilled in the art.

### <EXAMPLE 1: Synthesis and Physicochemical Characterization of Phenylcyclopropyl-Substituted Isoindolinone Derivative Compound Having Glutarimide Moiety>

The compound represented by Chemical Formula 1 of the present disclosure was prepared according to the methods as illustrated in Reaction Schemes 1 and 2:

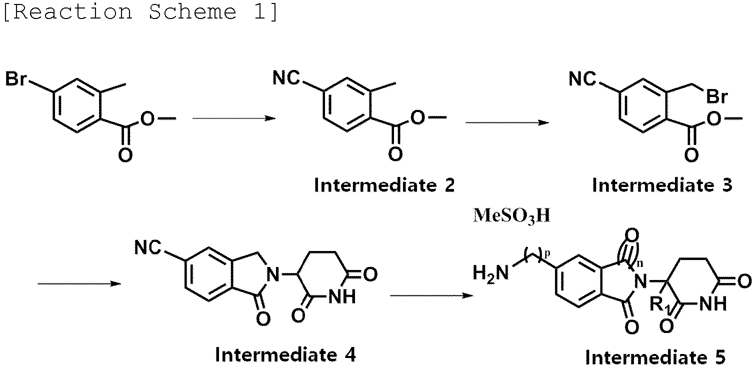

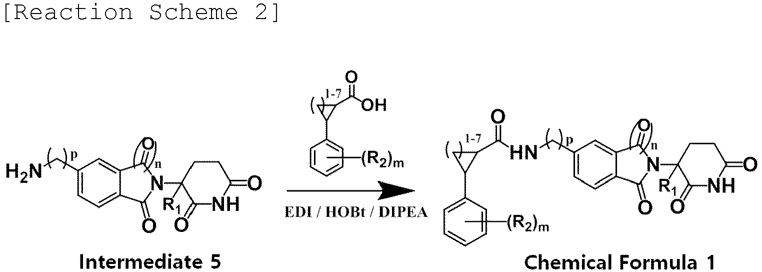

The preparation of intermediates 2 to 5 in Reaction Scheme 1 may be achieved as follows:

### 1) Synthesis of methyl 4-cyano-2-methylbenzoate (Intermediate 2)

To a solution of methyl 4-bromo-2-methylbenzoate (3 g, 13.10 mmol) in DMF (40 mL) were added Pd₂(dba)₃ (0.480 g, 0.524 mmol), dppf (0.436 g, 0.786 mmol), and zinc cyanide (1.692 g, 14.41 mmol) which were then stirred under nitrogen for 30 minutes before being heated at 130°C for 3 hours. After completion of the reaction, the reaction mixture was filtered through celite and washed several times with DMF. The filtrate was extracted with EtOAc (50 mL x 2) and washed with water and brine. The organic layer thus pooled was dried over anhydrous magnesium sulfate and concentrated under reduced pressure to remove the solvent. The concentrate was purified by column chromatography (Hexane : EtOAc = 9:1) to afford intermediate 2 as a bright yellow solid (1.96 g, 11.19 mmol, 85% yield).

### 2) Synthesis of methyl 2-(bromomethyl)-4-cyanobenzoate (Intermediate 3)

To a solution of Intermediate 2 (1.96 g, 11.19 mmol) in DCE (30 mL) were added N-bromosuccinimide (2.64 g, 22.38 mmol) and benzoyl peroxide (0.271 g, 1.119 mmol) that were stirred under reflux for 8 hours. After completion of the reaction, the reaction mixture was cooled to room temperature and concentrated under reduced pressure. The concentrate was purified by column chromatography (Hexane:EtOAc = 9:1) to afford Intermediate 3 as a yellow solid (2.29 g, 9.03 mmol, 81% yield).

### 3) Synthesis of 2-(2,6-dioxopiperidin-3-yl)-1-oxoindoline-5-carbonitrile (Intermediate 4)

To a solution of Intermediate 3 (7.34 g, 28.9 mmol) in DMF (50 mL) were added 3-aminopiperidine-2,6-dione hydrochloride (4.75 g, 28.9 mmol) and TEA (12.07 mL, 87 mmol) at room temperature. The mixture was stirred at 80°C overnight. After completion of the reaction, the reaction mixture was cooled to room temperature and concentrated under reduced pressure to remove the solvent. Water was added and the solid thus formed was filtered and washed with excess water. The solid was dried under reduced pressure to afford Intermediate 4 as a dark navy solid (6.28 g, 23.32 mmol, 81% yield).

### 4) Synthesis of 3-(5-(aminomethyl)-1-oxoisoindolin-2-yl)piperidine-2,6-dione (Intermediate 5)

To a solution of Intermediate 4 (1.7 g, 6.31 mmol) in DMA (20 ml) were added 10% Pd/C (2.32 g, 6.31 mmol) and methanesulfonic acid (0.451 ml, 6.94 mmol) which were then stirred overnight at 40°C under a hydrogen atmosphere. After completion of the reaction, the reaction mixture was filtered through celite and washed with 50 mL of DMA. The filtrate was concentrated under reduced pressure, and precipitation was induced using a methanol/ethyl ether (5 ml/30 ml) mixture. The precipitate thus formed was filtered to afford Intermediate 5 as a gray solid (0.99 g, 2.69 mmol, 43% yield).

¹H NMR (500 MHz, DMSO-*d*⁶) δ 11.00 (s, 1H), 8.27 (s, 3H), 7.79 (d, *J* = 7.8 Hz, 1H), 7.70 (s, 1H), 7.60 (d, *J* = 7.9 Hz, 1H), 5.12 (dd, *J* = 13.3, 5.1 Hz, 1H), 4.49 (d, *J* = 17.4 Hz, 1H), 4.35 (d, *J* = 17.4 Hz, 1H), 4.17 (q, *J* = 5.9 Hz, 2H), 2.97-2.87 (m, 1H), 2.60 (d, *J* = 17.4 Hz, 1H), 2.45-2.38 (m, 1H), 2.34 (s, 6H), 2.06-1.97 (m, 1H).

Compounds 1 to 14 of the present disclosure were prepared with reference to the method disclosed in Korean Patent No. 10-2011-0019761 A, and their physicochemical properties are as follows.

### Compound 1. (1S,2S)-N-((2-(2,6-dioxopiperidin-3-yl)-1-oxoisoindolin-5-yl)methyl)-2-phenylcyclopropane-1-carboxamide

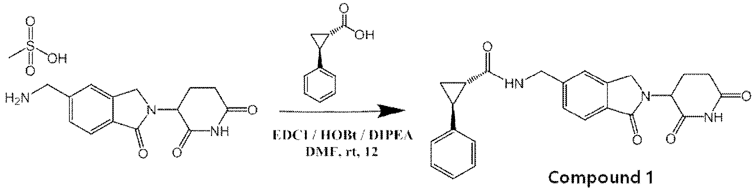

To a solution of 3-(5-(aminomethyl)-1-oxoisoindolin-2-yl)piperidine-2,6-dione methanesulfonate (15 mg, 0.00406 mmol) in DMF (1 mL) were added (1R,2R)-2-phenylcyclopropane-1-carboxylic acid (9.5 mg, 0.0487 mmol), EDCI·HCl (8.5 mg, 0.0446 mmol), HOBt·H₂O (6.0 mg, 0.0446 mmol), and DIPEA (28 µL, 0.162 mmol), which were then was stirred at room temperature for 12 hours. The reaction mixture was diluted with water before extraction with ethyl acetate. The organic layer was washed with brine, dried over anhydrous sodium sulfate, and filtered. The filtrate was concentrated under reduced pressure, and the concentrate was purified by column chromatography to afford Compound 1 as a white solid (5.0 mg, 28% yield).

¹H NMR (500 MHz, DMSO) δ 11.00 (s, 1H), 8.75 (t, J = 6.0 Hz, 1H), 7.70 (d, J = 7.9 Hz, 1H), 7.48 (s, 1H), 7.41 (d, J = 7.8 Hz, 1H), 7.28 (t, J = 7.5 Hz, 2H), 7.21 - 7.12 (m, 3H), 5.12 (dd, J = 13.3, 5.1 Hz, 1H), 4.50 - 4.36 (m, 3H), 4.31 (dd, J = 17.3, 2.8 Hz, 1H), 2.92 (m, 1H), 2.66 - 2.57 (m, 1H), 2.45 - 2.35 (m, 1H), 2.30 (m, 1H), 2.01 (m, 1H), 1.94 (m, 1H), 1.40 (m, 1H), 1.25 (m, 1H).

### Compound 2. (1R,2R)-N-((2-(2,6-dioxopiperidin-3-yl)-1-oxoisoindolin-5-yl)methyl)-2-phenylcyclopropane-1-carboxamide

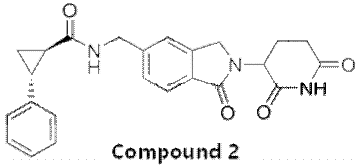

Compound 2 was synthesized in the same manner as for Compound 1 with the exception of using (1R,2R)-2-phenylcyclopropane-1-carboxylic acid (CAS no. : 3471-10-1) instead of (1R,2R)-2-phenylcyclopropane-1-carboxylic acid.

¹H NMR (400 MHz, DMSO) δ 10.98 (s, 1H), 8.74 (t, J = 5.9 Hz, 1H), 7.69 (d, J = 7.8 Hz, 1H), 7.48 (s, 1H), 7.41 (d, J = 7.8 Hz, 1H), 7.27 (m, 2H), 7.16 (m, 3H), 5.11 (dd, J = 13.3, 5.1 Hz, 1H), 4.44 (m, 3H), 4.31 (d, J = 17.2 Hz, 1H), 2.92 (m, 1H), 2.60 (m, 1H), 2.39 (m, 1H), 2.30 (m, 1H), 2.02 - 1.90 (m, 2H), 1.40 (m, 1H), 1.24 (m, 1H).

### Compound 3. (1R,2R)-2-(4-chlorophenyl)-N-((2-(2,6-dioxopiperidin-3-yl)-1-oxoisoindolin-5-yl)methyl)cyclopropane-1-carboxamide

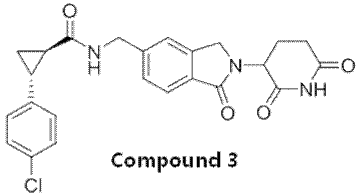

Compound 3 was synthesized in the same manner as for Compound 1 with the exception of using (1R,2R)-2-(4-chlorophenyl)cyclopropane-1-carboxylic acid (CAS no. : 31501-86-7) instead of (1R,2R)-2-phenylcyclopropane-1-carboxylic acid.

¹H NMR (400 MHz, DMSO) δ 10.98 (s, 1H), 8.75 (t, J = 6.0 Hz, 1H), 7.69 (d, J = 7.8 Hz, 1H), 7.48 (s, 1H), 7.37 (m, 3H), 7.18 (d, J = 8.1 Hz, 2H), 5.11 (dd, J = 13.2, 5.1 Hz, 1H), 4.55 - 4.23 (m, 4H), 2.92 (m, 1H), 2.60 (m, 1H), 2.46 - 2.27 (m, 2H), 2.07 - 1.87 (m, 2H), 1.40 (m, 1H), 1.25 (m, 1H).

### Compound 4. (1R,2S)-N-((2-(2,6-dioxopiperidin-3-yl)-1-oxoisoindolin-5-yl)methyl)-2-phenylcyclopropane-1-carboxamide

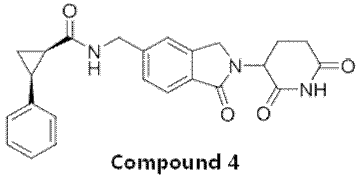

Compound 4 was synthesized in the same manner as for Compound 1 with the exception of using (1R,2S)-2-phenylcyclopropane-1-carboxylic acid(CAS no. : 939-89-9) instead of (1R,2R)-2-phenylcyclopropane-1-carboxylic acid.

¹H NMR (400 MHz, DMSO) δ 10.98 (s, 1H), 8.54 (t, J = 6.2 Hz, 1H), 7.53 (dd, J = 8.1, 2.7 Hz, 1H), 7.21 (m, 5H), 7.05 (m, 2H), 5.10 (dd, J = 13.2, 5.1 Hz, 1H), 4.34 (m, 2H), 4.16 (m, 2H), 2.92 (m, 1H), 2.70 - 2.56 (m, 1H), 2.46 - 2.38 (m, 2H), 2.11 (m, 1H), 2.03 - 1.95 (m, 1H), 1.58 (q, J = 5.9 Hz, 1H), 1.21 (m, 1H).

### Compound 5. (1R,2R)-2-(4-chlorophenyl)-N-((2-(2,6-dioxopiperidin-3-yl)-1-oxoisoindolin-5-yl)methyl)cyclopropane-1-carboxamide

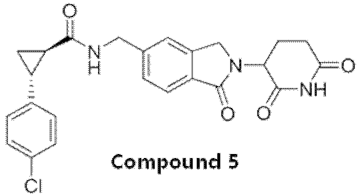

Compound 5 was synthesized in the same manner as for Compound 1 with the exception of using (1R,2R)-2-(4-chlorophenyl)cyclopropane-1-carboxylic acid(CAS no. : 4157-47-5) instead of (1R,2R)-2-phenylcyclopropane-1-carboxylic acid.

¹H NMR (400 MHz, DMSO) δ 10.98 (s, 1H), 8.75 (t, J = 5.9 Hz, 1H), 7.69 (d, J = 7.8 Hz, 1H), 7.48 (s, 1H), 7.40 (d, J = 7.9 Hz, 1H), 7.33 (d, J = 8.1 Hz, 2H), 7.18 (d, J = 8.1 Hz, 2H), 5.11 (dd, J = 13.5, 5.0 Hz, 1H), 4.62 - 4.20 (m, 4H), 3.00 - 2.82 (m, 1H), 2.60 (m, 1H), 2.47 - 2.28 (m, 2H), 2.05 - 1.84 (m, 2H), 1.41 (m, 1H), 1.26 (m, 1H).

### Compound 6. (1R,2R)-2-(3-chlorophenyl)-N-((2-(2,6-dioxopiperidin-3-yl)-1-oxoisoindolin-5-yl)methyl)cyclopropane-1-carboxamide

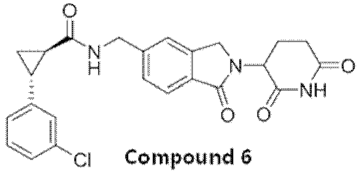

Compound 6 was synthesized in the same manner as for Compound 1 with the exception of using (1R,2S)-2-(3-chlorophenyl)cyclopropane-1-carboxylic acid(CAS no. : 16633-45-7) instead of (1R,2R)-2-phenylcyclopropane-1-carboxylic acid.

¹H NMR (400 MHz, DMSO) δ 10.98 (s, 1H), 8.74 (t, J = 6.0 Hz, 1H), 7.69 (d, J = 7.8 Hz, 1H), 7.48 (s, 1H), 7.41 (d, J = 7.9 Hz, 1H), 7.30 (t, J = 7.8 Hz, 1H), 7.24 (m, 2H), 7.13 (d, J = 7.6 Hz, 1H), 5.11 (dd, J = 13.3, 5.1 Hz, 1H), 4.53 - 4.25 (m, 4H), 2.92 (m, 1H), 2.60 (m, 1H), 2.44 - 2.29 (m, 2H), 2.00 (m, 2H), 1.41 (m, 1H), 1.30 (m, 1H).

### Compound 7. (1S,2S)-N-((2-(2,6-dioxopiperidin-3-yl)-1-oxoisoindolin-5-yl)methyl)-2-phenylcyclopropane-1-carboxamide

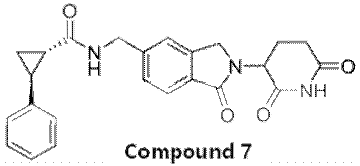

Compound 7 was synthesized in the same manner as for Compound 1 with the exception of using (1S,2S)-2-phenylcyclopropane-1-carboxylic acid(CAS no. : 23020-15-7) instead of (1R,2R)-2-phenylcyclopropane-1-carboxylic acid.

¹H NMR (400 MHz, DMSO) δ 10.98 (s, 1H), 8.74 (t, J = 6.0 Hz, 1H), 7.69 (d, J = 7.8 Hz, 1H), 7.48 (s, 1H), 7.41 (d, J = 7.8 Hz, 1H), 7.28 (m, 2H), 7.21 - 7.09 (m, 3H), 5.11 (dd, J = 13.4, 5.0 Hz, 1H), 4.51 - 4.23 (m, 4H), 2.92 (m, 1H), 2.67 - 2.55 (m, 1H), 2.45 - 2.22 (m, 2H), 2.07 - 1.87 (m, 2H), 1.40 (m, 1H), 1.24 (m, 1H).

### Compound 8. (1R,2R)-N-((2-(2,6-dioxopiperidin-3-yl)-1-oxoisoindolin-5-yl)methyl)-2-(4-fluorophenyl)cyclopropane-1-carboxamide

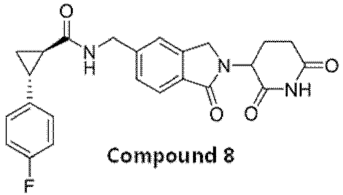

Compound 8 was synthesized in the same manner as for Compound 1 with the exception of using (1R,2R)-2-(4-fluorophenyl)cyclopropane-1-carboxylic acid(CAS no. : 515179-19-8) instead of (1R,2R)-2-phenylcyclopropane-1-carboxylic acid.

¹H NMR (400 MHz, DMSO) δ 10.99 (s, 1H), 8.74 (t, J = 5.9 Hz, 1H), 7.69 (d, J = 7.8 Hz, 1H), 7.48 (s, 1H), 7.41 (d, J = 7.9 Hz, 1H), 7.19 (m, 2H), 7.14 - 7.06 (m, 2H), 5.11 (dd, J = 13.4, 4.9 Hz, 1H), 4.44 (m, 3H), 4.31 (d, J = 17.3 Hz, 1H), 2.92 (m, 1H), 2.65 - 2.55 (m, 1H), 2.46 - 2.37 (m, 1H), 2.31 (m, 1H), 2.00 (m, 1H), 1.90 (m, 1H), 1.38 (m, 1H), 1.23 (m, 1H).

### Compound 9. (1R,2R)-N-((2-(2,6-dioxopiperidin-3-yl)-1-oxoisoindolin-5-yl)methyl)-2-(3-fluorophenyl)cyclopropane-1-carboxamide

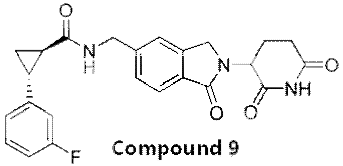

Compound 9 was synthesized in the same manner as for Compound 1 with the exception of using (1R,2R)-2-(3-fluorophenyl)cyclopropane-1-carboxylic acid(CAS no. : 175168-72-6) instead of (1R,2R)-2-phenylcyclopropane-1-carboxylic acid.

¹H NMR (400 MHz, DMSO) δ 10.99 (s, 1H), 8.74 (t, J = 5.9 Hz, 1H), 7.69 (d, J = 7.8 Hz, 1H), 7.48 (s, 1H), 7.41 (d, J = 7.9 Hz, 1H), 7.35 - 7.26 (m, 1H), 7.05 - 6.96 (m, 3H), 5.11 (dd, J = 13.3, 4.9 Hz, 1H), 4.44 (m, 3H), 4.31 (d, J = 17.3 Hz, 1H), 3.02 - 2.82 (m, 1H), 2.65 - 2.56 (m, 1H), 2.44 - 2.27 (m, 2H), 2.00 (m,, 2H), 1.41 (m, 1H), 1.35 - 1.26 (m, 1H).

### Compound 10. (1S,2S)-2-(3-chlorophenyl)-N-((2-(2,6-dioxopiperidin-3-yl)-1-oxoisoindolin-5-yl)methyl)cyclopropane-1-carboxamide

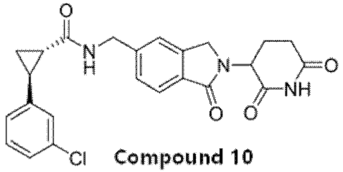

Compound 10 was synthesized in the same manner as for Compound 1 with the exception of using (1S,2S)-2-(3-chlorophenyl)cyclopropane-1-carboxylic acid (CAS no. : 16633-45-7) instead of (1R,2R)-2-phenylcyclopropane-1-carboxylic acid.

¹H NMR (400 MHz, DMSO) δ 10.99 (s, 1H), 8.74 (t, J = 6.0 Hz, 1H), 7.69 (d, J = 7.8 Hz, 1H), 7.48 (s, 1H), 7.41 (d, J = 7.9 Hz, 1H), 7.30 (t, J = 7.8 Hz, 1H), 7.27 - 7.21 (m, 2H), 7.13 (d, J = 7.7 Hz, 1H), 5.11 (dd, J = 13.4, 5.0 Hz, 1H), 4.42 (m, 3H), 4.31 (d, J = 17.3 Hz, 1H), 2.99 - 2.82 (m, 1H), 2.63 (m, 1H), 2.45 - 2.27 (m, 2H), 2.06 - 1.96 (m, 2H), 1.42 (m, 1H), 1.30 (m, 1H).

### Compound 11. N-((2-(2,6-dioxopiperidin-3-yl)-1-oxoisoindolin-5-yl)methyl)-2-(4-(trifluoromethyl)phenyl)cyclopropane-1-carboxamide

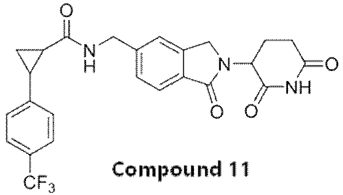

Compound 11 was synthesized in the same manner as for Compound 1 with the exception of using Racemic-(1R,2R)-2-(3-chlorophenyl)cyclopropane-1-carboxylic acid(CAS no. : 2262-03-5) instead of (1R,2R)-2-phenylcyclopropane-1-carboxylic acid.

¹H NMR (400 MHz, DMSO) δ 10.99 (s, 1H), 8.78 (t, J = 6.0 Hz, 1H), 7.69 (d, J = 7.6 Hz, 1H), 7.63 (d, J = 8.0 Hz, 2H), 7.48 (s, 1H), 7.39 (m, 3H), 5.11 (dd, J = 13.5, 4.9 Hz, 1H), 4.45 (m, 3H), 4.31 (d, J = 17.3 Hz, 1H), 2.92 (m, 1H), 2.60 (m, 1H), 2.39 (m, 2H), 2.07 - 1.94 (m, 2H), 1.47 (m, J = 4.5 Hz, 1H), 1.35 (q, J = 4.3, 3.3 Hz, 1H).

### Compound 12. (1S,2S)-N-((2-(2,6-dioxopiperidin-3-yl)-1-oxoisoindolin-5-yl)methyl)-2-(p-tolyl)cyclopropane-1-carboxamide

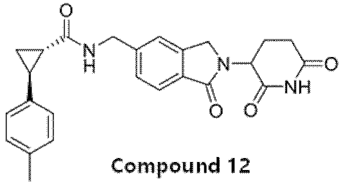

Compound 12 was synthesized in the same manner as for Compound 1 with the exception of using (1S,2R)-2-(p-tolyl)cyclopropane-1-carboxylic acid(CAS no. : 16633-44-6) instead of (1R,2R)-2-phenylcyclopropane-1-carboxylic acid.

¹H NMR (400 MHz, DMSO) δ 10.99 (s, 1H), 8.72 (m, 1H), 7.69 (d, J = 7.9 Hz, 1H), 7.48 (s, 1H), 7.40 (d, J = 7.9 Hz, 1H), 7.08 (d, J = 7.8 Hz, 2H), 7.02 (d, J = 7.9 Hz, 2H), 5.11 (dd, J = 13.3, 5.0 Hz, 1H), 4.41 (m, 3H), 4.31 (d, J = 17.3 Hz, 1H), 2.98 - 2.84 (m, 1H), 2.64 - 2.55 (m, 1H), 2.45 - 2.31 (m, 1H), 2.25 (m, 4H), 2.04 - 1.95 (m, 1H), 1.88 (m, 1H), 1.36 (m, 1H), 1.22 - 1.16 (m, 1H).

### Compound 13. 2-(2,4-dichlorophenyl)-N-((2-(2,6-dioxopiperidin-3-yl)-1-oxoisoindolin-5-yl)methyl)cyclopropane-1-carboxamide

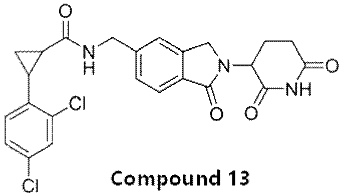

Compound 13 was synthesized in the same manner as for Compound 1 with the exception of using Racemic-2-(2,4-dichlorophenyl)cyclopropane-1-carboxylic acid instead of (1R,2R)-2-phenylcyclopropane-1-carboxylic acid.

¹H NMR (400 MHz, DMSO) δ 10.99 (s, 1H), 8.79 (t, J = 6.0 Hz, 1H), 7.69 (d, J = 7.8 Hz, 1H), 7.62 (m,1H), 7.49 (s, 1H), 7.44 - 7.36 (m, 2H), 7.18 (d, J = 8.4 Hz, 1H), 5.12 (dd, J = 13.4, 4.9 Hz, 1H), 4.54 - 4.39 (m, 3H), 4.31 (d, J = 17.2 Hz, 1H), 2.92 (m, 1H), 2.61 (m, 1H), 2.49 - 2.37 (m, 2H), 2.06 - 1.95 (m, 1H), 1.87 (m, 1H), 1.39 (m, 2H).

### Compound 14. N-((2-(2,6-dioxopiperidin-3-yl)-1-oxoisoindolin-5-yl)methyl)-2-phenylcyclobutane-1-carboxamide

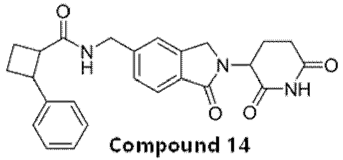

Compound 14 was synthesized in the same manner as for Compound 1 with the exception of using Racemic-2-phenylcyclobutane-1-carboxylic acid (CAS no. : 91142-51-0) instead of (1R,2R)-2-phenylcyclopropane-1-carboxylic acid.

¹H NMR (400 MHz, DMSO) δ 10.99 (s, 1H), 8.42 (m, 0.5 H), 8.06 (m, 0.5 H), 7.67 (d, J = 7.9 Hz, 0.5 H), 7.49 (s, J = 7.7 Hz, 0.5 H), 7.36 (d, J = 7.8 Hz, 0.5 H), 7.31 (m, 1H), 7.22 (m, 4H), 6.87 (m, 1H), 5.11 (m, 1H), 4.50 - 4.16 (m, 4H), 3.97 (m, 1H), 3.89 (m, 0.5H), 3.68 (m, 0.5H), 3.44 (m, 1H), 3.10 (m, 1H), 2.92 (m, 1H), 2.64 - 2.55 (m, 1H), 2.41 (m, 1H), 2.29 - 1.91 (m, 5H).

### <COMPARATIVE EXAMPLE 1. Synthesis and Physicochemical Characterization of Comparative Isoindolinone Derivatives Having Glutamimide Moiety>

Physicochemical properties of Comparative Compounds 1 to 3 are as follows.

### Comparative Compound 1. N-((2-(2,6-dioxopiperidin-3-yl)-1-oxoisoindolin-5-yl)methyl)isoquinoline-3-caboxamide

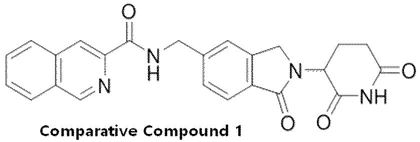

Comparative Compound 1 was synthesized in the same manner as for Compound 1 with the exception of using isoquinoline-3-carboxylic acid instead of 6-chloro-1H-indazole-3-carboxylic acid.

¹H NMR (500 MHz, DMSO-d ₆) δ 10.98 (s, 1H), 9.62 (t, J = 6.4 Hz, 1H), 9.41 (s, 1H), 8.59 (s, 1H), 8.27 (d, J = 8.1 Hz, 1H), 8.20 (d, J = 8.2 Hz, 1H), 7.89 (t, J = 7.5 Hz, 1H), 7.85 - 7.79 (m, 1H), 7.69 (d, J = 7.8 Hz, 1H), 7.58 (s, 1H), 7.51 (d, J = 7.9 Hz, 1H), 5.10 (dd, J = 13.3, 5.1 Hz, 1H), 4.68 (d, J = 6.4 Hz, 2H), 4.44 (d, J = 17.3 Hz, 1H), 4.30 (d, J = 17.3 Hz, 1H), 2.98 - 2.84 (m, 1H), 2.59 (m, 1H), 2.37 (m, 1H), 2.03 - 1.92 (m, 1H).

### Comparative Compound 2. N-((2-(2,6-dioxopiperidin-3-yl)-1-oxoisoindolin-5-yl)methyl)quinolxaline-2-carboxamide

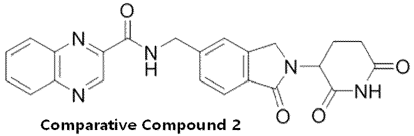

Comparative Compound 2 was synthesized in the same manner as for Compound 1 with the exception of using quinoxaline-2-carboxylic acid instead of 6-chloro-1H-indazole-3-carboxylic acid.

¹H NMR (500 MHz, DMSO-d ₆) δ 10.98 (s, 1H), 9.77 (t, J = 6.4 Hz, 1H), 9.50 (s, 1H), 8.22 (m, 2H), 8.04 - 7.98 (m, 2H), 7.70 (d, J = 7.8 Hz, 1H), 7.61 (s, 1H), 7.54 (d, J = 7.9 Hz, 1H), 5.10 (dd, J = 13.3, 5.1 Hz, 1H), 4.69 (d, J = 6.4 Hz, 2H), 4.44 (d, J = 17.4 Hz, 1H), 4.31 (d, J = 17.3 Hz, 1H), 2.91 (m, 1H), 2.59 (m, 1H), 2.43 - 2.32 (m, 1H), 2.06 - 1.95 (m, 1H).

### Comparative Compound 3. 6-chloro-N-((2-(2,6-dioxopiperidin-3-yl)-1-oxoisoindolin-5-yl)methyl)quinoline-2-carboxamide

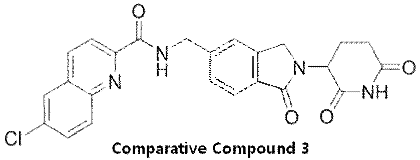

Comparative Compound 3 was synthesized in the same manner as for Compound 1 with the exception of using 6-chloroquinoline-2-carboxylic acid instead of 6-chloro-1H-indazole-3-carboxylic acid.

¹H NMR (300 MHz, DMSO) δ 10.98 (s, 1H), 9.65 (t, J = 6.4 Hz, 1H), 8.57 (d, J = 8.5 Hz, 1H), 8.27 (d, J = 2.4 Hz, 1H), 8.23 (d, J = 8.6 Hz, 1H), 8.16 (d, J = 9.1 Hz, 1H), 7.90 (dd, J = 9.0, 2.4 Hz, 1H), 7.71 (d, J = 7.8 Hz, 1H), 7.60 (s, 1H), 7.53 (dd, J = 7.8, 1.4 Hz, 1H), 5.11 (dd, J = 13.2, 5.1 Hz, 1H), 4.69 (d, J = 6.3 Hz, 2H), 4.45 (d, J = 17.4 Hz, 1H), 4.31 (d, J = 17.4 Hz, 1H), 2.99 - 2.84 (m, 1H), 2.59 (m, 1H), 2.35 (m, 1H), 2.06 - 1.94 (m, 1H).

### EXPERIMENTAL EXAMPLE 1. Evaluation of Substrate Protein Degradation Activity against CRBN

To determine whether the compounds of the present disclosure specifically bind to CRBN (cereblon) to inhibit its function, investigation was made of the effect of the compounds on the degradation of Ikaros (IKZF1) protein (Chamberlain, P. P. *et al.,* 2014) or GSPT1 protein (Matyskiela, M. E. *et al.,* 2016), which are known to be degraded by CRBN upon binding to thalidomide and analogs thereof.

To evaluate the degradation activity of the compounds of the present disclosure on Ikaros (IKZF1) protein or GSPT1 protein, the following experiment was conducted.

KG-1 cells were seeded at a density of 5x10⁵ cells per well in a 12-well plate and each compound was added at predetermined concentrations. After 6 hours, cell lysates were collected using TBSN buffer. The compounds were assessed for degradation activity by Western blot analysis using an anti-Ikaros antibody against Ikaros protein and using an anti-GSPT1 antibody against GSPT1. To this end, equal amounts of protein were loaded into each well of a 4-15% gradient gel, and after electrophoresis, proteins were transferred to a PVDF membrane. The membrane was probed with primary antibodies against each target protein, followed by HRP-conjugated secondary antibodies and detection using an HRP substrate.

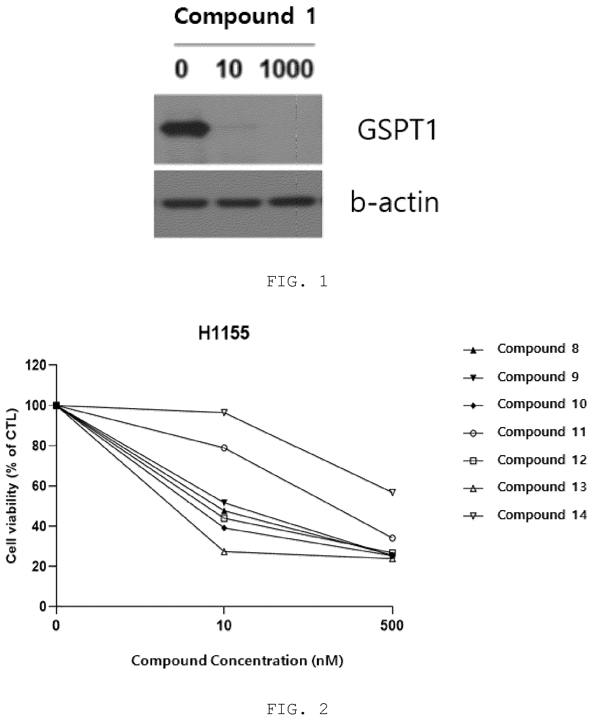

As shown in FIG. 1, the compound of the present disclosure selectively promoted degradation of GSPT1 protein after 6 hours of treatment in the KG-1 cell line.

### EXPERIMENTAL EXAMPLE 2. Cytotoxicity Assay

To evaluate the effects of the compounds of the present disclosure on cancer cells, a cytotoxicity assay was conducted as follows.

Cancer cells (KG-1) were seeded at a density of 10,000 cells per well in 96-well plates. The compound of the present disclosure and control compounds (Comparative Compounds 1, 2, and 3) were treated at predetermined concentrations. After 72 hours, the cells were treated with WST-1 reagent for 1 hour. Then, absorbance was read at 450 nm using a SpectraMax spectrophotometer to determine cell viability. Based on the absorbance values, IC₅₀ (µM) values were calculated using GraphPad Prism and are shown in Table 1.

The viability of NCI-H1155 (lung cancer cells) was measured using the CytoX cell viability assay kit (LPS Solution, #CYT3000). NCI-H1155 cells were seeded in a 12-well plate, and each compound of the present disclosure and a comparative compound (CC-90009) were treated at 10 nM and 500 nM concentrations for 72 hours. CytoX solution was then added to the cells and incubated for 1 hour. Absorbance was read at 450 nm on a microplate reader, and % viability was calculated and shown in Table 1.

As shown in FIG. 2, the compound of the present disclosure exhibited excellent growth inhibitory effects on NCI-H1155 lung cancer cells.

**TABLE 1**

| Compound No. | Cytotoxicity KG-1 (IC₅₀, µM) | NCI-H1155 (% viability) | |
|---|---|---|---|
| | | at 10 nM | at 500 nM |
| Cpd. 1 | 0.002 | nt | nt |
| Cpd. 2 | nt | 5 | 5 |
| Cpd. 3 | nt | 4 | 4 |
| Cpd. 4 | nt | 94 | 95 |
| Cpd. 5 | nt | 2 | 4 |
| Cpd. 6 | nt | 5 | 4 |
| Cpd. 7 | nt | 3 | 4 |
| Cpd. 8 | nt | 48 | 26 |
| Cpd. 9 | nt | 52 | 25 |
| Cpd. 10 | nt | 39 | 25 |
| Cpd. 11 | nt | 79 | 34 |
| Cpd. 12 | nt | 44 | 27 |
| Cpd. 13 | nt | 27 | 24 |
| Cpd. 14 | nt | 96 | 57 |
| Comparative Cpd. 1 | > 10 | nt | |
| Comparative Cpd. 2 | > 10 | nt | |
| Comparative Cpd. 3 | 4.1 | nt | |
| CC-90009 (Eragidomide) | nt | 85.0 | 23.25 |
| nt ; not tested | | | |

As shown in Table 1, the phenylcyclopropane-substituted isoindolinone derivatives having a glutarimide moiety according to the present disclosure exhibited superior cytotoxicity against KG-1 cancer cells compared to the comparative compounds. Compounds 1 to 13, having a phenylcyclopropane moiety as a substituent, exhibited better cytotoxicity against cancer cells than Compound 14, which has a phenylcyclobutane moiety as a substituent. In addition, with respect to the geometric isomerism for the substituent phenylcyclopropane moiety, the trans-isomers (Compounds 1-3 and 5-10) showed better cytotoxicity against NCI-H1155 cancer cells than the cis-isomer compound (Compound 4).

### FORMULATION EXAMPLE 1. Preparation of Powder

Two g of Compound 1 of the present disclosure was mixed with 1 g of lactose and the mixture was loaded into an airtight packet to prepare a powder formulation.

### FORMULATION EXAMPLE 2. Preparation of Tablet

One hundred mg of Compound 1 of the present disclosure was mixed with 100 mg of microcrystalline cellulose, 60 mg of lactose monohydrate, 20 mg of low-substituted hydroxypropyl cellulose, and 2 mg of magnesium stearate, and the mixture was compressed to prepare a tablet according to a conventional method for preparing tablets.

### FORMULATION EXAMPLE 3. Preparation of Capsule

One hundred mg of Compound 1 of the present disclosure was mixed with 100 mg of microcrystalline cellulose, 60 mg of lactose monohydrate, 20 mg of low-substituted hydroxypropyl cellulose, and 2 mg of magnesium stearate. The mixture was loaded into a gelatin capsule to prepare a capsule formulation.

### FORMULATION EXAMPLE 4. Preparation of Pill

Ninety mg of Compound 1 of the present disclosure was mixed with 5 mg of glutinous rice starch, 5 mg of purified water, and small amounts of additives that inhibit hygroscopicity, such as dextrin, maltodextrin, corn starch, and microcrystalline cellulose (MCC). Pills of 100 mg were prepared according to a conventional method.

### FORMULATION EXAMPLE 5. Preparation of Injection

Ten mg of Compound 1 of the present disclosure was mixed with a sufficient amount of sterile distilled water for injection and a pH adjusting agent. The mixture was prepared into ampoules (2 mL per ampoule) according to conventional methods for injection preparation.

## Claims

1. A compound represented by the following Chemical Formula 1, or a racemate, an optical isomer, a stereoisomer, or a pharmaceutically acceptable salt thereof: 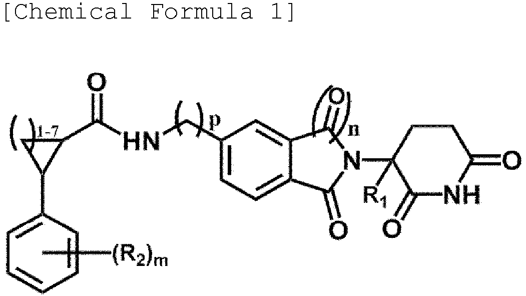 wherein,
m is an integer from 0 to 3;
n is an integer of 0 or 1;
p is an integer of 1 or 2;
R₁ is hydrogen or deuterium (D);
R₂ is independently at least one substituent selected from the group consisting of hydrogen, halogen, hydroxy, carboxylic acid, C₁₋₅ alkylcarboxyl, amino, acetamido, sulfonic acid, C₁₋₅ alkylsulfonic acid, nitro, mono- or di-(C₁₋₅ alkyl) amino, C₁₋₅ alkyl, C₁₋₃ alkoxy, halo-C₁₋₃ alkyl, and halo-C₁₋₃ alkoxy.

2. The compound, or racemate, optical isomer, stereoisomer, or pharmaceutically acceptable salt thereof as set forth in claim 1, wherein the compound represented by Chemical Formula 1 is a compound represented by the following Chemical Formula 2: 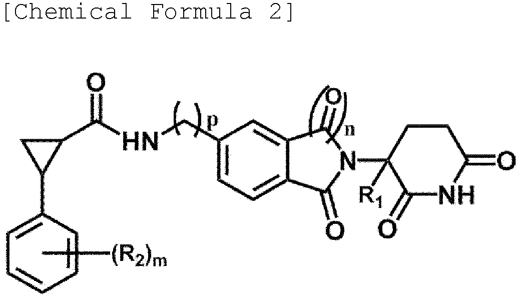 wherein,
m is an integer from 0 to 3;
p is an integer of 1 or 2;
R₁ is hydrogen or deuterium (D);
R₂ is independently at least one substituent selected from the group consisting of hydrogen, halogen, hydroxy, carboxylic acid, C₁₋₅ alkylcarboxyl, amino, acetamido, sulfonic acid, C₁₋₅ alkylsulfonic acid, nitro, mono- or di-(C₁₋₅ alkyl) amino, C₁₋₅ alkyl, C₁₋₃ alkoxy, halo-C₁₋₃ alkyl, and halo-C₁₋₃ alkoxy.

3. The compound, or racemate, optical isomer, or pharmaceutically acceptable salt thereof as set forth in claim 1, wherein the compound represented by Chemical Formula 1 is selected from the group consisting of:
(1S,2S)-N-((2-(2,6-dioxopiperidin-3-yl)-1-oxoisoindolin-5-yl)methyl)-2-phenylcyclopropane-1-carboxamide (Compound 1);
(1R,2R)-N-((2-(2,6-dioxopiperidin-3-yl)-1-oxoisoindolin-5-yl)methyl)-2-phenylcyclopropane-1-carboxamide (Compound 2);
(1R,2R)-2-(4-chlorophenyl)-N-((2-(2,6-dioxopiperidin-3-yl)-1-oxoisoindolin-5-yl)methyl)cyclopropane-1-carboxamide (Compound 3);
(1R,2S)-N-((2-(2,6-dioxopiperidin-3-yl)-1-oxoisoindolin-5-yl)methyl)-2-phenylcyclopropane-1-carboxamide (Compound 4);
(1R,2R)-2-(4-chlorophenyl)-N-((2-(2,6-dioxopiperidin-3-yl)-1-oxoisoindolin-5-yl)methyl)cyclopropane-1-carboxamide (Compound 5);
(1R,2R)-2-(3-chlorophenyl)-N-((2-(2,6-dioxopiperidin-3-yl)-1-oxoisoindolin-5-yl)methyl)cyclopropane-1-carboxamide (Compound 6);
(1S,2S)-N-((2-(2,6-dioxopiperidin-3-yl)-1-oxoisoindolin-5-yl)methyl)-2-phenylcyclopropane-1-carboxamide (Compound 7);
(1R,2R)-N-((2-(2,6-dioxopiperidin-3-yl)-1-oxoisoindolin-5-yl)methyl)-2-(4-fluorophenyl)cyclopropane-1-carboxamide (Compound 8);
(1R,2R)-N-((2-(2,6-dioxopiperidin-3-yl)-1-oxoisoindolin-5-yl)methyl)-2-(3-fluorophenyl)cyclopropane-1-carboxamide (Compound 9);
(1S,2S)-2-(3-chlorophenyl)-N-((2-(2,6-dioxopiperidin-3-yl)-1-oxoisoindolin-5-yl)methyl)cyclopropane-1-carboxamide (Compound 10);
N-((2-(2,6-dioxopiperidin-3-yl)-1-oxoisoindolin-5-yl)methyl)-2-(4-(trifluoromethyl)phenyl)cyclopropane-1-carboxamide (Compound 11);
(1S,2S)-N-((2-(2,6-dioxopiperidin-3-yl)-1-oxoisoindolin-5-yl)methyl)-2-(p-tolyl)cyclopropane-1-carboxamide (Compound 12);
2-(2,4-dichlorophenyl)-N-((2-(2,6-dioxopiperidin-3-yl)-1-oxoisoindolin-5-yl)methyl)cyclopropane-1-carboxamide (Compound 13); and
N-((2-(2,6-dioxopiperidin-3-yl)-1-oxoisoindolin-5-yl)methyl)-2-phenylcyclobutane-1-carboxamide (Compound 14); or
a pharmaceutically acceptable salt thereof.

4. A pharmaceutical composition comprising the compound represented by Chemical Formula 1 or a pharmaceutically acceptable salt thereof according to claim 1 as an active ingredient for prevention or treatment of leprosy, chronic graft-versus-host disease, inflammatory disease, or cancer.

5. The pharmaceutical composition according to Claim 4, wherein the cancer is selected from the group consisting of breast cancer, colon cancer, lung cancer, small cell lung cancer, gastric cancer, liver cancer, hematologic cancer, bone cancer, pancreatic cancer, skin cancer, head or neck cancer, cutaneous or ocular melanoma, uterine cancer, ovarian cancer, rectal cancer, anal cancer, colorectal cancer, fallopian tube carcinoma, endometrial carcinoma, cervical cancer, vaginal cancer, vulvar carcinoma, Hodgkin's disease, esophageal cancer, small intestine cancer, endocrine gland cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, CNS tumors, primary CNS lymphoma, spinal cord tumor, brainstem glioma, and pituitary adenoma.
